# EUROPEAN PATENT SPECIFICATION

(11) **EP 0 685 207 B1**
(45) Date of publication and mention of the grant of the patent: **17.04.2002**
(21) Application number: 95106886.5
(22) Date of filing: 06.05.1995
(51) Int. Cl.: A61B 17/70

(54) **A device for stabilizing or, respectively, compressing or distracting portions of the spinal column**
Vorrichtung zum Stabilisieren bzw. Komprimieren oder Distrahieren von Abschnitten der Wirbelsäule
Dispositif pour stabiliser les parties du rachis, ou pour les mettre sous compression ou tension

(30) Priority: 04.06.1994 DE 9409123 U
(43) Date of publication of application: 06.12.1995
(60) Divisional application: 01103156.4
(73) Proprietor: Howmedica GmbH, D-24232 Schönkirchen (DE)
(72) Inventor: Metz-Stavenhagen, Peter, Dr., D-34537 Bad Wildungen (DE); Robioneck, Bernd, D-24211 Preetz (DE)
(74) Representative: Patentanwälte Hauck, Graalfs, Wehnert, Döring, Siemons

(56) References cited:
- EP-A- 0 328 883
- WO-A-93/15697
- US-A- 4 987 892
- US-A- 5 047 029

## Description

The present invention relates to a device for stabilizing or respectively compressing or distracting certain portions of the spinal column, according to the preamble of claim 1.

Implants utilizing a so-called distracting rod for correcting the spinal column are conventional.
French patent 2 289 164 as well as British patent 2 131 300 disclose hooks to be provided at the ends of a distracting rod engaging beyond the pedicels of the respective vertebra. Still further, German 33 06 657 discloses a distracting rod variable in length and to be utilized together with pedicel screws. The pedicel screws are screwed into pedicels of the respective vertebra and the annular head is slid onto a diminished end of the distracting rod to be fixed thereto by means of a nut.

EP 0 159 007 teaches a pedicel screw including a plate-shaped projection having a serration cooperating with a serrated surface at the end of a distracting rod.

As disclosed in EP 0 452 792, a threaded rod inserted through the annular-shaped head of a pedicel screw is fixed to the pedicel screw head by means of nuts.

According to WO 90/09 156 the head of a pedicel screw is provided with a slot receiving a distracting rod. By means of a fixing screw secured to the head, the distracting rod may be fixed to the head of the pedicel screw.

Pedicel screws are often utilized in devices for stabilizing adjacent vertebras (for example EP 0 558 883). In this case they cannot be utilized at the same time to hold a distraction rod. In order to transmit certain forces through a distracting rod, it must have a minimum diameter which, however, is in many cases larger than the usual inner diameter of an annular-shaped head of a pedicel screw.

WO-A-93 15697 discloses a device for stabilizing and/or compressing or distracting sections of the spinal column wherein a threaded bolt is inserted through the hole of the pedicel screw head. The head of the bolt has a through-going opening for receiving a distraction rod. The rectangular head of the bolt is extending through a washer between the rod and the pedicel screw head. The washer has a bore complementary to the cross section of the bolt head. The washer further has a splined surface cooperating with the splined surface of the pedicel screw head.

US-A-4 987 892 discloses a device for stabilizing sections of the human spine wherein a clamping portion is provided which is approximately U-shaped with the legs having a through-bore and the outer side of one leg having a splined surface adapted to cooperate with the splined surface of the pedicel screw head. The connecting portion of the clamping portion has a through-going passage adapted to receive a distraction rod. A screw bolt is inserted through the bores in the legs of the clamping portion and the screw head to press the parts against each other, with the distraction rod received by the clamping portion being clampingly fixed.

It is the object of the present invention to provide a device for stabilizing or, respectively, compressing or distracting portions of the spinal column to which the pedicel screws may be applied, which is versatile and provides a connection to a distracting rod at the same time.

According to the invention, the object referred to is solved by the features of claim 1.

Accordingly, the device of the present invention utilizes known pedicel screws including an annular-shaped head, wherein at least one side of the head includes a roughened portion. The roughened portion comprises an annular serration, for example, which is conventional. According to the invention, an adapter is provided which may be connected by screw means to the head of the pedicel screw, wherein a roughened portion of the adapter cooperates with the roughened portion of the head to provide for a non-rotatable connection of the adapter with respect to the pedicel screw. The adapter has further a circular receiving opening or a receiving recess for receiving the threaded rod which is fixed to the adapter by threaded means. The receiving opening or recess being spaced from the pedicel screw head.

The device according to the invention provides for the advantage that conventional pedicel screws of standard dimensions may be utilized while a threaded rod of sufficient thickness may be used. There is the further advantage that connecting the rod to the pedicel screws is obtained in a simple fashion as the rod is first mounted in the adapter which is then secured to the head of the pedicel screw. If an adapter must be placed through the head of a pedicel screw, problems might occur when the pedicel screw, or, respectively the head thereof is not aligned with respect to the axis of the threaded rod which is movable to a limited extent.

Preferably the adapter is dimensioned to be relatively short, including substantially two portions such as a mounting portion to be connected to the pedicel screw head and a receiving portion having an opening for receiving the threaded rod. Still further, the adapter is shaped such that the axis of the bore receiving the threaded rod is normal to the axis of the pedicel screw head.

The adapter includes a threaded shank portion to be inserted through the head of the pedicel screw and to be secured by means of a nut screwed on the threaded portion. The receiving portion includes a roughened portion cooperating with the roughened portion of the pedicel screw head.

According to an embodiment of the invention, the adapter is secured to the threaded rod by two nuts each one located at either side of the adapter. According to another embodiment, the threaded rod has a flattened portion or the like to non-rotatably, but axially slidably receiving a washer including a roughened portion at one side thereof which is urged towards the roughened portion of the adapter by the nut. The threaded rod preferably includes a pair of reversed threaded portions and a tool-engaging profile, preferably centrally located to exert a tensioning action when rotating the threaded rod. Accordingly, adapters located at opposite ends of the rod may be biased apart from each other or, respectively, towards each other in order to provide respective distracting or compressing forces.

Referring now to the drawings:
- Fig. 1: shows an embodiment of a device according to the invention;
- Fig. 2: shows an adapter for the device of Fig. 1; and
- Fig. 3: shows the connection of the device of Fig. 1 and 2 to a threaded rod.

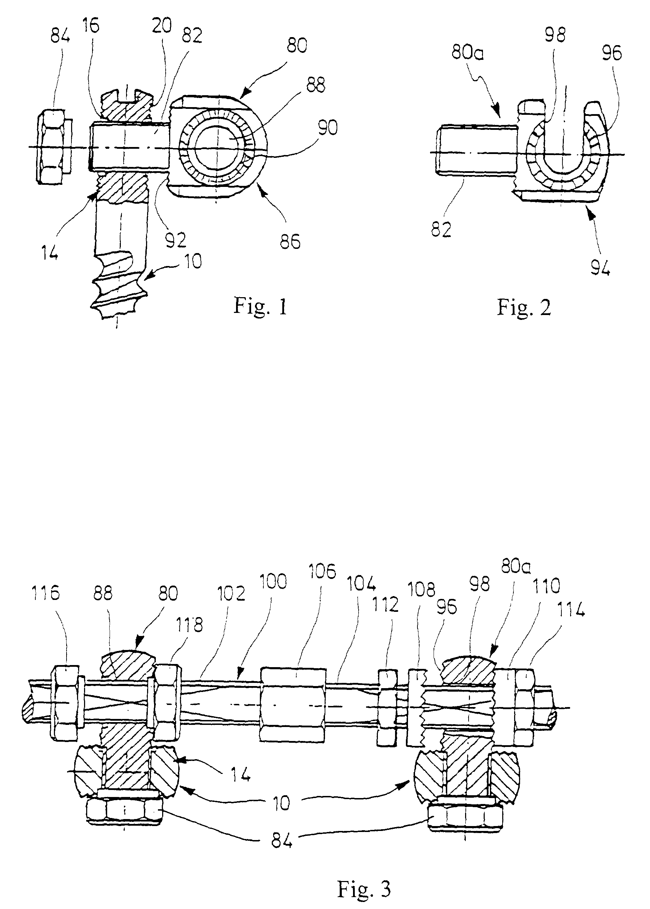

Fig. 1 shows a conventional pedicel screw 10 having a screw shank 12 and an annular head 14 including a circular opening 16 as well as serrated faces on opposite sides as seen at 18 and 20.

Furthermore, Fig. 1 shows an adapter 80 having a threaded shank portion 82 which is inserted to the opening 16 of the head 14 of the pedicel screw 10 to be secured to the pedicel screw head 14 by means of a nut 84. The nut has an axial flange engaging the enlarged portion of the opening 16.

The receiving portion 86 of the adapter 80 includes a receiving opening 88 provided for a threaded rod extending therethrough which opening is provided with a serrated annular face 90. The axis of the opening 88 is located normal with respect to the opening 16 in the head 14. The receiving portion 86 has a serrated annular face 92 cooperating with the serrated annular face 20 or, respectively 18 of the screw head 14 as many be seen in Fig. 3.

Fig. 2 shows an adapter 80a having a receiving portion 94 which is fork-shaped and has an annular face section 96 including a serration corresponding to the serrated annular 90 shown in Fig. 1. The recess has the reference numeral 98.

According to the embodiment shown in Fig. 3, the threaded rod 100 is provided with threaded portion 102, 104 reverse with respect to each other, wherein a central hexagonal portion 106 is provided which may be engaged by a tool for rotating the rod 100 (for distracting or compressing). The portion 102, 104 of the rod are provided with flattened portions. Washers 108, respectively 110 having serrated head faces are non-rotatably arranged on the threaded portion 104 to the right end of the rod, any may be secured to the correspondingly serrated annular faces 96 of the adapter 80a by means of nuts 112, 114. The adapter 80 shown in Fig. 3 is connected to the rod 100 merely by means of the nuts 116, 118 having axial flanges cooperating with an enlarged portion within the opening 88.

As may be seen, the threaded rod extends under a right angle with respect to the axis of the opening in the pedicel screw head. The adapter is shaped such that the threaded rod is located relatively close to the head of the pedicel screw to provide a rather compact arrangement.

## Claims

1. A device for stabilizing or, respectively, compressing or distracting sections of the spinal column, comprising
- at least a pair of pedicle screws, the pedicle screws (10) having a shank (12) and an annular head (14), the annular head (14) having a roughened surface portion (20) on at least one face thereof which extends parallel to the axis of the shank (12),
- adapter means (80) for each pedicle screw (10) having a receiving portion and mounting means, the mounting means having a threaded shank portion (82, 82a) adapted to be inserted through the head (14) of the pedicle screw (10) and to be secured by means of a nut (84) screwed on said threaded shank portion,
- the mounting means of the adapter means having a roughened portion (92) cooperating with the roughened surface portion (20) of the annular head (14) to counteract a rotation of the adapter means with respect to the pedicle screw,
- the receiving portion of the adapter having a circular receiving bore (88) for receiving a rod (100) to be extended therethrough, the rod being adapted to be secured to the adapter means by threaded clamping means,
**characterized in that**
- an integral adapter is provided as adapter means,
- the receiving portion of the adapter being provided with two parallel spaced surfaces extending around the receiving bore at opposite ends of the bore, each of the two surfaces being provided with a second roughened portion (90),
- the roughened portion of the mounting means being formed by a roughened surface of the receiving portion so that the annular head of the pedicle screw is clamped between the roughened surface of the receiving portion and the nut on the shank of the mounting portion, and
- the rod has a thread and the threaded clamping means include separate nut means (112, 114, 116, 118) adapted to be secured onto the rod,
- wherein each nut means is acting on one of the second roughened portions.

2. The device of claim 1, wherein the threaded rod (100) includes portions (102, 104) having reverse threaded portions and wherein the threaded rod (100) is provided with tool engaging faces (106).

3. The device of claim 1 or 2, wherein the threaded rod (62, 76, 100) includes a flattened portion (64) for non-rotatably receiving a washer (66, 68) which has a roughened portion at one side which is to coact with the second roughened portion.

## Patentansprüche

1. Vorrichtung zum Stabilisieren bzw. zum Zusammenpressen oder zur Distraktion von Abschnitten der Wirbelsäule, mit
- mindestens einem Paar Stielschrauben, wobei die Stielschrauben (10) einen Schaft (12) und einen ringförmigen Kopf (14) aufweisen und der ringförmige Kopf (14) auf mindestens einer Seite desselben einen gerauhten Flächenabschnitt (20) aufweist, der parallel zu der Achse des Schafts (12) verläuft,
- ein Adaptermittel (80) für jede Stielschraube (10) mit einem Aufnahmeabschnitt und einem Befestigungsmittel, wobei das Befestigungsmittel einen durch den Kopf (14) der Stielschraube (10) hindurch einführbaren und mit Hilfe einer auf den mit Gewinde versehenen Schaftabschnitt geschraubten Mutter (84) sicherbaren, mit Gewinde versehenen Schaftabschnitt (82, 82a) aufweist,
- wobei das Befestigungsmittel des Adaptermittels einen gerauhten Abschnitt (92) aufweist, der mit dem gerauhten Flächenabschnitt (20) des ringförmigen Kopfes (14) zusammenwirkt, um einer Drehung des Adaptermittels in bezug auf die Stielschraube entgegenzuwirken,
- wobei der Aufnahmeabschnitt des Adapters eine kreisförmige Aufnahmebohrung (88) zum Aufnehmen eines durch diese hindurchzuführenden Stabs (100) aufweist, wobei der Stab durch mit Gewinde versehene Spannmittel an dem Adapter gesichert werden kann,
**dadurch gekennzeichnet, daß**
- ein einstückig ausgebildeter Adapter als Adaptermittel vorgesehen ist,
- der Aufnahmeabschnitt des Adapters mit zwei parallel beabstandeten, sich an einander gegenüberliegenden Enden der Bohrung um die Aufnahmebohrung herum erstreckenden Flächen versehen ist, wobei jede der zwei Flächen mit einem zweiten gerauhten Abschnitt (90) versehen ist,
- der gerauhte Abschnitt des Befestigungsmittels durch eine gerauhte Fläche des Aufnahmeabschnitts gebildet wird, so daß der ringförmige Kopf der Stielschraube zwischen der gerauhten Fläche des Aufnahmeabschnitts und der Mutter auf dem Schaft des Befestigungsabschnitts gespannt wird, und
- der Stab ein Gewinde aufweist und die mit Gewinde versehenen Spannmittel getrennte, auf dem Stab sicherbare Muttern (112, 114, 116, 118) umfassen,
- wobei jede Mutter auf einen der zweiten gerauhten Abschnitte einwirkt.

2. Vorrichtung nach Anspruch 1, wobei der Gewindestab (100) Abschnitte (102, 104) mit Gegengewindebereichen umfaßt, und wobei der Gewindestab (100) mit Werkzeugeingriffsflächen (106) versehen ist.

3. Vorrichtung nach Anspruch 1 oder 2, wobei der Gewindestab (62, 76, 100) einen abgeflachten Abschnitt (64) zum nicht drehbaren Aufnehmen einer Unterlegscheibe (66, 68) umfaßt, die an einer Seite, die mit dem zweiten gerauhten Abschnitt zusammenwirken soll, einen gerauhten Abschnitt aufweist.

## Revendications

1. Dispositif destiné à stabiliser ou, respectivement, comprimer ou écarter des sections de la colonne vertébrale, comprenant
- au moins une paire de vis pédiculaires, les vis pédiculaires (10) comprenant une tige (12) et une tête annulaire (14), la tête annulaire (14) présentant une partie de surface rendue rugueuse (20) sur au moins une face de celle-ci qui s'étend parallèlement à l'axe de la tige (12),
- un moyen d'adaptateur (80) pour chaque vis pédiculaire (10) comprenant une partie de réception et un moyen de montage, le moyen de montage comprenant une partie de tige filetée (82, 82a) conçue pour être insérée au travers de la tête (14) de la vis pédiculaire (10) et pour être fixée au moyen d'un écrou (84) vissé sur ladite partie de tige filetée,
- le moyen de montage du moyen d'adaptateur présentant une partie rendue rugueuse (92) coopérant avec la partie de surface rendue rugueuse (20) de la tête annulaire (14) afin de contrecarrer une rotation du moyen d'adaptateur par rapport à la vis pédiculaire,
- la partie de réception de l'adaptateur présentant un alésage de réception circulaire (88) destiné à recevoir une tige (100) devant s'étendre au travers de celui-ci, la tige étant conçue pour être fixée au moyen d'adaptateur par des moyens de serrage filetés,
**caractérisé en ce que**
- un adaptateur intégral est prévu en tant que moyen d'adaptateur,
- la partie de réception de l'adaptateur étant munie de deux surfaces espacées parallèles s'étendant autour de l'alésage de réception aux extrémités opposées de l'alésage, chacune des deux surfaces étant munie d'une seconde partie rendue rugueuse (90),
- la partie rendue rugueuse du moyen de montage étant formée par une surface rendue rugueuse de la partie de réception de sorte que la tête annulaire de la vis pédiculaire soit serrée entre la surface rendue rugueuse de la partie de réception et l'écrou sur la tige de la partie de montage, et
- la tige comporte un filet et le moyen de serrage fileté comprend un moyen d'écrou séparé (112, 114, 116, 118) conçu pour être fixé sur la tige,
- dans lequel chaque moyen d'écrou agit sur l'une des secondes parties rendues rugueuses.

2. Dispositif selon la revendication 1, dans lequel la tige (100) comprend des parties (102, 104) présentant des parties filetées en sens inverse et dans lequel la tige filetée (100) est munie de faces de mise en prise d'outil (106).

3. Dispositif selon la revendication 1 ou 2, dans lequel la tige filetée (62, 76, 100) comprend une partie aplatie (64) destinée à recevoir sans possibilité de rotation une rondelle (66, 68) qui présente une partie rendue rugueuse d'un côté laquelle doit agir de concert avec la seconde partie rendue rugueuse.
